# EUROPÄISCHE PATENTSCHRIFT

(11) **EP 0 596 903 B1**
(45) Veröffentlichungstag und Bekanntmachung des Hinweises auf die Patenterteilung: **23.04.1997**
(21) Anmeldenummer: 92914236.2
(22) Anmeldetag: 07.07.1992
(51) Int. Cl.: A61K 9/70

(54) **TRANSDERMALE THERAPEUTISCHE SYSTEME**
TRANSDERMAL THERAPEUTIC SYSTEMS
SYSTEMES THERAPEUTIQUES TRANSDERMIQUES

(30) Priorität: 30.07.1991 DE 4125611; 25.03.1992 DE 4210165
(43) Veröffentlichungstag der Anmeldung: 18.05.1994
(73) Patentinhaber: SCHERING AKTIENGESELLSCHAFT, 13342 Berlin (DE)
(72) Erfinder: RIEDL, Jutta, D-1000 Berlin 19 (DE); LIPP, Ralph, D-1000 Berlin 31 (DE); HARTISCH, Matthias, D-13359 Berlin (DE)
(86) Internationale Anmeldenummer: EP9201515
(87) Internationale Veröffentlichungsnummer: WO9302669

(56) Entgegenhaltungen:
- EP-A- 0 091 964
- EP-A- 0 224 981
- EP-A- 0 370 220
- EP-A- 0 399 432
- EP-A- 0 417 496
- EP-A- 0 430 491
- WO-A-91/05529
- PHARMACEUTICAL TECHNOLOGY, September 1990, Seiten 132-140, PFISTER, W.R. und HSIEH, D.S.T.

## Beschreibung

Die Erfindung betrifft transdermale therapeutische Systeme, die in einer wirkstoffhaltigen Klebematrix einen polaren Penetrationsverstärker und einen in diesem unlöslichen unpolaren Penetrationsverstärker enthalten.

Transdermale therapeutische Systeme (TTS) sind bekanntlich mehrschichtig aufgebaute wirkstoffhaltige Pflaster, die auf der Haut fixiert werden und die ihren Wirkstoff perkutan über einen längeren Zeitraum kontinuierlich abgeben. Um eine therapeutisch ausreichende Penetrationsgeschwindigkeit des Wirkstoffes durch die Haut zu erzielen, ist es meist erforderlich, die transdermalen therapeutischen Systeme zusätzlich zum Wirkstoff mit Penetrationsverstärkern zu versehen.

Transdermale therapeutische Systeme, die eine Kombination eines polaren Pentetrationsverstärker und einem in diesem unlöslichen unpolaren Penetrationsverstärker enthalten sind vorbekannt. So werden in der EP-A-0 430 491, in der EP-A-0 399 432, in der EP-A-0 224 981 und in der WO 91/05529 Systeme beschrieben, die neben einem unpolaren Penetrationsverstärker als unpolaren Penetrationsverstärker Alkandiole enthalten.

Es wurde nun gefunden, daß es überraschenderweise möglich ist, transdermale therapeutische Systeme herzustellen, die in einer wirkstoffhaltigen Klebermatrix einen polaren Penetrationsverstärker und einen in diesem unlöslichen unpolaren Penetrationsverstärker enthalten, welche dadurch gekennzeichnet sind, daß der polare Penetrationsverstärker Harnstoff ist.

Die so hergestellten erfindungsgemäßen transdermalen therapeutischen Systeme haben gegenüber vergleichbaren Systemen die in einer Matrix entweder nur einen polaren Penetrationsverstärker oder nur einen unpolaren Penetrationsverstärker enthalten mehrere Vorteile:
1. Die Verwendung der kombinierten Penetrationsverstärker bewirkt oft eine unerwartet starke Steigerung der Penetrationsgeschwindigkeit des Wirkstoffes.
2. Die Verwendung der speziellen Kombination führt oft dazu, daß die Beladungsobergrenzen der einzelnen Penetrationsverstärker überschritten werden können.
3. Eine schlechte Haftung des Systems auf der Haut, wie sie bei transdermalen Systemen beobachtet wird, die an der Beladungsobergrenze mit einem polaren Penetrationsverstärker beladen sind, wird bei den erfindungsgemäßen Systemen nicht beobachtet.
4. Pflasterrückstände, wie sie nach der Applikation von Systemen, die unpolare Penetrationsverstärker enthalten oft beobachtet werden, treten nicht auf.

Insgesamt werden durch die erfindungsgemäße Kombination die Klebeeigenschaften der transdermalen Systeme verbessert und ihr Tragekomfort durch Abpuffern der Feuchte während des Tragens erhöht.

Als Matrix bildenden Kleber enthält das erfindungsgemäße System vorzugsweise einen käuflich erwerbbaren Kleber (Pharmaceutical Technology, 1989, p 126-138). Derartige Kleber sind beispielsweise Polyacrylatkleber des Durotak® -Typs (National Starch. Comp.), Sichello® -Typs (Sichelwerke) oder Gelva® -Typs (Monsanto Comp.), Synthesekautschukkleber des Adhesin® -Typs (Henkel KG) oder Silikonklebers des X7-Typs (Dow Corning Corp.).

Als unpolaren Penetrationsverstärker enthalten die erfindungsgemäßen transdermalen Systeme vorzugsweise einen Fettsäureester mit mindestens 8 Kohlenstoffatomen.

Fettsäureester, die sich für das erfindungsgemäße Verfahren eignen sind beispielsweise solche der Laurinsäure, Myristinsäure, Palmitinsäure, Isopalmitinsäure Stearinsäure und Isostearinsäure, wie zum Beispiel die Methylester, Ethylester, Propylester, Isopropylester, Butylester, sec.-Butylester, Isobutylester, tert.-Butylester oder Glycerinester.

Solche pharmazeutisch üblichen Fettsäureester sind zum Beispiel das Methylmyristat, das Ethylmyristat, das Propylmyristat, das Methyllaurat, das Ethyllaurat, das Propyllaurat, das Isopropyllaurat, das Methyloleat, das Ethyloleat, das Propyloleat, das Isopropyloleat und das Isopropylpalmitat und insbesondere das Isopropylmyristat. Sicherlich dürften auch hochsiedende flüssige Kohlenwasserstoffe mit mit mindestens 8 Kohlenstoffatomen und Gemische dieser Kohlenwasserstoffe, wie Paraffinum liquidum oder Terpenkohlenwasserstoffe, wie α-Pinen, d-Limonen, 3-Caren, Myrcen, Camphen, Bisabolen oder Squalen, Alkohole mit mindestens 8 Kohlenstoffatomen, wie zum Beispiel die Alkanole, Myristylalkohol oder Stearylalkohol oder Sterine, wie Cholesterol und auch Fettsäuren mit mindestens 8 Kohlenstoffatomen, wie die Stearinsäure, Palmitinsäure, Ölsäure zur Herstellung der erfindungsgemäßen transdermalen Systeme geeignet sein. Versuche, daß dieses zutrifft liegen zur Zeit noch nicht vor.

Der unpolare Penetrationsverstärker wird vorzugsweise so dosiert, daß seine Konzentration in der fertigen Matrix 1 bis 15 Gewichtsprozent bezogen auf diese beträgt.

Wirkstoffe die sich zur Herstellung der erfindungsgemäßen transdermalen Systeme eignen sind vorzugsweise solche, die in Wasser schwer löslich oder unlöslich sind. Geeignet sind zum Beispiel Steroidhormone wie:

Gestagen wirksame Steroidhormone, wie beispielsweise das 13-Ethyl-17β-hydroxy-18,19-dinor-17α-pregn-4-en-20yl-3-on (=Levonorgestrel), das 13-Ethyl-17β-hydroxy-18,19-dinor-17α-pregna-4,15-dien-20yn-3-on (=Gestoden) oder das 13-Ethyl-17β-hydroxy-11-methylen-18,19-dinor-17α-pregn-4-en-20yn (=Desorgestrel), Estrogen wirksame Steroidhormone das 3-Hydroxy-1,3,5-(10)-estratrien-17-on (=Estron), das 1,3,5(10)-Estratrien-3,17β-diol (=Estradiol) oder das 1,9-Nor-17α-pregna-1,3,5(10)-thien-20yn-3,17β-diol (=Ethinylestradiol).

Androgen wirksame Steroidhormone wie das 17β-Hydroxy-4-androsten-3-on (=Testosteron) und dessen Ester oder das 17β-Hydroxy-1α-methyl-5α-androsten-3-on (=Mesterolon).

Antiandrogen wirksame Steroidhormone wie das 17α-Acetoxy-6-chlor-1β,2β-dihydro-3H-cyclopropa[1,2]-pregna-1,4,6-trien-3,20-dion (=Cypoteronacetat).

Kortikoide wie das 11β,17α,21-Trihydroxy-4-pregnen-3,20-dion (=Hydrocortison), das 11β,17α,21-Trihydroxy-1,4-pegnadien-3,20-dion (=Prednisolon), das 11β,17α,21-Trihydroxy-6α-methyl-1,4-pregnatrien-3,20-dion (=Methylprednisolon) und das 6α-Fluor-11β,21-dihydroxy-16α-methyl-1,4-pregnadien-3,20-dion (=Diflucortolon) und deren Ester.

### Geeignete Wirkstoffe sind ferner:

Ergolin-Derivate, wie das Lisurid, [=3-(9,10-Didehydro-6-methyl-8α-ergolinyl)-1,1-diethylharnstoff],das Bromlisurid [=3-(2-Brom-9,10-dehydro-6-methyl-8α-ergolinyl-1,1-diethylharnstoff] das Tergurid [=3-(6-Methyl-8α-ergolinyl-1,1-diethylharnstoff] und das Protergurid [=3-(6-Propyl-8α-ergolinyl)-1,1-diethylharnstoff].

Antihypertonika wie das 7α-Acetylthio-17α-hydroxy-3-oxo-4-pregnen-21-carbonsäure-γ-lacton (= Spironolacton) und das 7α-Acetylthio-15β-,16β-methylen-3-oxo-17α-pregna-1,4-dien-21,17-carbolacton (=Mespirenon).

Antikoagulantia wie die 5-[Hexahydro-5-hydroxy-4-(3-hydroxy-4-methyl-1-octen-6-ynyl)-2(1H)-pentalenyliden)]-pentansäure (=Iloprost) oder die (Z)-7-[(1R,2R,3R,5R)-5-Chlor-3-hydroxy-2-[(E)-(3R)-3-hydroxy-4,4-dimethyl-1-octenyl]-cyclopentyl]-5-heptensäure (=Nocloprost).

Psychopharmaka wie das 4-(3-Cyclopentyloxy-4-methoxy-pheny-2-pyrrolidon (=Rolipram) und das 7-Chlor-1,3-dihydro-1-methyl-5-phenyl-2H-1,4-benzodiazepin-2-on (=Diazepam).

Organische Nitroverbindungen, wie das Nitroglycerin oder das Isosorbiddinitrat [=1,4,3,6-Dianhydro-D-glucitol-dinitrat].

Betablocker, wie das Propanolol {= 1-[(1-Methylethyl)-amino]-3-(1-naphthyloxy-2-propanolol}, das Mepindolol {= 1-[(1-Methylethyl)-amino]-3-[(2-methyl-1H-inol-4-yl)-oxy]-2-propanol} und das Carazolol {= 2-(9H-Carbazol-4-yloxy)-3-[(1-methethyl)-amino]-2-propanol}.

Carotinoide wie das α-Carotin und das β-Carotin.

Eine weitere Gruppe sind β-Carboline, wie sie beispielsweise in den Europäischen Patentanmeldungen 234,173 und 239,667 beschrieben sind. Als β-Carboline seien beispielsweise genannt, der 6-Benzoyloxy-4-methoxymethyl-β-carbolin-3-carbonsäure-isopropylester (=Becarnil) und der 5-(4-Chlorphenoxy)-4-methoxymethyl-β-carbolin-3-carbonsäure-isopropylester (=Cl-PHOCIP).

Erwähnenswert sind auch Analgetika, wie zum Beispiel die Salicylsäure, das Glycolsalicylat, das Methylsalycilat, das 7,8-Didehydro-4,5-epoxy-17-methyl-morphinan-3,6-diol (=Morphin), das 4,5-Epoxy-14-hydroxy-3-methoxy-17-methyl-morphinan-6-on (=Oxycodon), das (-)-(R)-6-(Dimethylaminol-4,4-diphenyl-3-heptanon (=Levomethadon) oder das 3,4,5,6-Tetrahydro-5-methyl-1-phenyl-1H-2,5-benzoxacin (=Nefopam).

Letztlich seien als geeignete Wirkstoffe das Nicotin, Clonidin und das Scopolamin erwähnt.

Es ist einleuchtend, daß die erfindungsgemäßen transdermalen Systeme auch Gemische dieser Wirkstoffe enthalten können.

Die optimale Konzentration des Wirkstoffs in den erfindungsgemäßen transdermalen therapeutischen Systemen ist naturgemäß von der Art des Wirkstoffs, seiner Wirksamkeit, der Art der Penetrationsverstärker, des verwendeten Klebers etc. abhängig und muß im Einzelfalle durch die dem Galeniker vertrauten Vorversuche ermittelt werden. In der Regel wird er den Wirkstoff so dosieren, daß seine Konzentration in der fertigen Matrix 0,1 bis 10 Gewichtsprozent bezogen auf diese beträgt.

Die erfindungsgemäßen transdermalen therapeutischen Systeme sind vorzugsweise so beschaffen, daß sie aus einer für die Penetrationsverstärker und gegebenenfalls auch für Wasser undurchlässigen Deckschicht, einer auf der Deckschicht haftenden wirkstoffhaltigen Klebematrix, welche den polaren und unpolaren Penetrationsverstärker enthält und einer abziehbaren Schutzschicht bestehen.

Diese einfachste Form eines transdermalen therapeutischen Systems, welches man als Klebermatrix-TTS bezeichnet, kann in der Weise hergestellt werden, daß man eine Lösung des Klebers in einem niedrigsiedenden Lösungsmittel mit dem Wirkstoff oder Wirkstoffgemisch, dem polaren und dem unpolaren Penetrationsverstärker mischt, die Mischung folienartig auf einer für die Penetrationsverstärker und gegebenenfalls auch für Wasser undurchlässigen Deckschicht aufträgt, das flüchtige Lösungsmittel durch Erwärmen entfernt und das erhaltene Produkt mit einer abziehbaren Schutzschicht abdeckt.

Geeignete Lösungsmittel zur Auflösung des Klebers sind beispielsweise niedrigsiedende Alkohole, wie Methanol, Ethanol oder Isopropanol, niedrigsiedende Ketone, wie Aceton, niedrigsiedende Kohlenwasserstoffe wie Hexan, oder niedrigsiedende Ester, wie Ethylacetat sowie Mischungen derselben.

Dieses Verfahren kann in der Weise durchgeführt werden, daß man eine Lösung oder Suspension des Wirkstoffs, der Penetrationsverstärker und des Klebers in einem flüchtigen Lösungsmittel auf eine plane undurchlässige Deckschicht gestrichen und nach dem Trocknen bei etwa 60° C bis 90° C mit einer abziehbaren Schutzschicht versehen wird.

Als Schutzschichten eignen sich alle Folien, die man üblicherweise bei transdermalen therapeutischen Systemen mit Kleber anwendet. Solche Folien sind beispielsweise silikonisiert oder fluorpolymerbeschichtet.

Als Deckschicht kann man bei diesem System beispielsweise 10 bis 100 µm dicke Folien aus Polyethylen, PVC, PVDC oder deren Kopolymerisate oder Polyester wahlweise pigmentiert oder metallisiert verwenden. Die hierauf aufgebrachte Arzneimittelschicht hat vorzugsweise eine Dicke von 20 bis 500 µm. Die Abgabe der Wirkstoffe erfolgt vorzugsweise über eine Fläche von 5 bis 100 cm².

Es ist für den Fachmann offenkundig, daß die erfindungsgemäßen transdermalen therapeutischen Systeme auch wesentlich komplexer gestaltet werden können als die bereits erwähnten einfachen Matrixsysteme. (Yie W. Chien: "Transdermal Controlled Systemic Medications", Marcel Dekker, Inc., New York and Basel, 1987, Dr. Richard Baker: "Analysis of Transdermal Drug Delivery Patents 1934 to 1984" und "Analysis of Recent Transdermal Delivery Patents, 1984-1986 and Enhancers" Membrane Technology & Research 1030 Hamilton Court Menlo Park CA 94025 (415) 328-2228). Dies dürfte aber in der Regel keinerlei nennenswerte Vorzüge der Systeme bringen, die den erhöhten Aufwand zu ihrer Herstellung rechtfertigen.

Die nachfolgenden Ausführungsbeispiele dienen zur näheren Erläuterung der Erfindung:

### Beispiel 1

Zu 100 g einer 50 gewichtsprozentigen Lösung von Acrylatkleber Gelva® (Hersteller: Monsanto Comp.) in Ethylacetat werden nacheinander
- 3,00 g: 17β-Estradiol
- 6,00 g: mikronisierter Harnstoff, dispergiert in Hexan und
- 3,00 g: Isopropylmyristat
gegeben. Die sich bildende trübe Masse wird anschließend in einem Edelstahlgefäß gerollt, um vorhandene Gasblasen zu entfernen.

Mittels einer Knife-over-Roll Beschichtungsvorrichtung wird die weitgehend gasblasenfreie Masse auf eine fluorpolymerbeschichtete Polyesterfolie (Scotchpack® 1022 der Firma 3 M, Minneapolis) oder eine silikonisierte Folie (Akrosil Silox B 54 der Firma Akrosil, Comp.) in der Weise aufgetragen, daß nach dem Entfernen des flüchtigen Lösungsmittels bei 65-75° C über 2 bis 3 Minuten ein gleichmäßiger Film von 50 g/m² entsteht. Anschließend wird mit einer Polyesterabdeckfolie (Hytrel 6108 Folie 30µm - der Firma Bertek, Vermont/USA kaschiert. Das so erhaltene Laminat wird mittels einer Stanzvorrichtung in Einzelpflaster von 5 cm², 10 cm² und 20 cm² Fläche geteilt und in aluminierte Beutel verpackt. Die Pflaster haften nach Abziehen der Schutz-Folie auf der Haut und können zur Hormonsubstitution verwendet werden.

### Beispiel 2

Zu 100 g einer 50 gewichtsprozentigen Lösung von Silikonkleber X7-4502 (Hersteller: Dow Corning Comp.) in Hexan werden nacheinander
- 3,00 g: 17β-Estradiol
- 5,00 g: mikronisierter Harnstoff, dispergiert in Hexan und
- 2,00 g: Isopropylmyristat
gegeben. Die sich bildende trübe Masse wird anschließend in einem Edelstahlgefäß gerollt, um vorhandene Gasblasen zu entfernen.

Mittels einer Knife-over-Roll Beschichtungsvorrichtung wird die weitgehend gasblasenfreie Masse auf eine fluorpolmerbeschichtete Polyesterfolie (Scotchpack® 1022 der Firma 3 M, Minneapolis) oder eine silikonisierte Folie (Akrosil Silox B 54 der Firma Akrosil, Comp.) in der Weise aufgetragen, daß nach dem Entfernen des flüchtigen Lösungsmittels bei 65-75° C über 2 bis 3 Minuten ein gleichmäßiger Film von 50 g/m² entsteht. Anschließend wird mit einer Polyesterabdeckfolie (Hytrel 6108 Folie 30µm - der Firma Bertek, Vermont/USA kaschiert. Das so erhaltene Laminat wird mittels einer Stanzvorrichtung in Einzelpflaster von 5 cm², 10 cm² und 20 cm² Fläche geteilt und in aluminierte Beutel verpackt. Die Pflaster haften nach Abziehen der Schutz-Folie auf der Haut und können zur Hormonsubstitution verwendet werden.

## Patentansprüche

1. Transdermale therapeutische Systeme, die in einer wirkstoffhaltigen Klebematrix einen polaren Penetrationsverstärker und einen in diesem unlöslichen unpolaren Penetrationsverstärker enthalten, dadurch gekennzeichnet, daß der polare Penetrationsverstärker Harnstoff ist.

2. Transdermale therapeutische Systeme gemäß Patentanspruch 1, dadurch gekennzeichnet, daß sie als unpolaren Penetrationsverstärker einen Kohlenwasserstoff, einen Alkohol, eine Fettsäure oder einen Fettsäureester mit jeweils mindestens 8 Kohlenstoffatomen enthalten.

3. Transdermale therapeutische Systeme gemäß Patentanspruch 2, dadurch gekennzeichnet, daß sie als Fettsäureester Isopropylmyristat enthalten.

4. Transdermale therapeutische Systeme gemäß Patentanspruch 1 bis 3, dadurch gekennzeichnet, daß sie aus einer für die Penetrationsverstärker und gegebenenfalls auch für Wasser undurchlässigen Deckschicht, einer auf der Deckschicht haftenden wirkstoffhaltigen Klebematrix, welche den polaren und unpolaren Penetrationsverstärker enthält und einer abziehbaren Schutzschicht bestehen.

5. Verfahren zur Herstellung transdermaler therapeutischer Systeme gemäß Patentanspruch 4, dadurch gekennzeichnet, daß man eine Lösung des Klebers in einem niedrigsiedenden Lösungsmittel mit dem Wirkstoff oder Wirkstoffgemisch, dem polaren und dem unpolaren Penetrationsverstärker mischt, die Mischung folienartig auf einer für die Penetrationsverstärker und gegebenenfalls auch für Wasser undurchlässigen Deckschicht aufträgt, das flüchtige Lösungsmittel durch Erwärmen entfernt und das erhaltene Produkt mit einer abziehbaren Schutzschicht abdeckt.

## Claims

1. Transdermal therapeutic systems that comprise in an active-ingredient-containing adhesive matrix a polar penetration promoter and a nonpolar penetration promoter that is insoluble in the polar penetration promoter, characterised in that the polar penetration promoter is urea.

2. Transdermal therapeutic systems according to patent claim 1, characterised in that they comprise as nonpolar penetration promoter a hydrocarbon, an alcohol, a fatty acid or a fatty acid ester, each having at least 8 carbon atoms.

3. Transdermal therapeutic systems according to patent claim 2, characterised in that they comprise isopropylmyristate as fatty acid ester.

4. Transdermal therapeutic systems according to patent claims 1 to 3, characterised in that they consist of a cover layer that is impermeable to the penetration promoters and optionally also to water, an active-ingredient-containing adhesive matrix that adheres to the cover layer, which adhesive matrix comprises the polar and nonpolar penetration promoters, and a removable protective layer.

5. Process for the preparation of transdermal therapeutic systems according to patent claim 4, characterised in that a solution of the adhesive in a low-boiling solvent is mixed with the active ingredient or active ingredient mixture and the polar and nonpolar penetration promoters, the mixture is applied in the manner of a foil to a cover layer that is impermeable to the penetration promoters and optionally also to water, the volatile solvent is removed by heating and the resulting product is covered by a removable protective layer.

## Revendications

1. Systèmes thérapeutiques transdermiques comprenant, dans une matrice de colle contenant le principe actif, un activateur de pénétration polaire et un activateur de pénétration non polaire insoluble dans l'activateur polaire, caractérisé en ce que l'activateur de pénétration polaire est l'urée.

2. Systèmes thérapeutiques transdermiques selon la revendication 1, caractérisés en ce qu'ils contiennent comme activateur de pénétration non polaire un hydrocarbure, un alcool, un acide gras ou un ester d'acide gras, chacun avec au moins 8 atomes de carbone.

3. Systèmes thérapeutiques transdermiques selon la revendication 2, caractérisés en ce qu'ils contiennent comme ester d'acides gras le myristate d'isopropyle.

4. Systèmes thérapeutiques transdermiques selon les revendications 1 à 3, caractérises en ce qu'ils sont constitués d'une couche extérieure imperméable aux activateurs de pénétration et éventuellement aussi à l'eau, d'une matrice de colle contenant le principe actif, adhérente à la couche extérieure, laquelle matrice contient les activateurs de pénétration polaire et non polaire, et d'une couche protectrice retirable.

5. Procédé pour la préparation de systèmes thérapeutiques transdermiques selon la revendication 4, caractérisé en ce que l'on mélange une solution de la colle dans un solvant à bas point d'ébullition avec le principe actif ou le mélange de principes actifs, avec les activateurs de pénétration polaire et non polaire, on applique le mélange sous forme de pellicule sur une couche extérieure imperméable aux activateurs de pénétration et éventuellement aussi à l'eau, on élimine par chauffage le solvant volatil et on recouvre le produit obtenu d'une couche protectrice retirable.
